# EUROPÄISCHE PATENTSCHRIFT

(11) **EP 3 370 682 B1**
(45) Veröffentlichungstag und Bekanntmachung des Hinweises auf die Patenterteilung: **18.09.2019**
(21) Anmeldenummer: 16782220.4
(22) Anmeldetag: 17.10.2016
(51) Int. Cl.: A61K 8/06, A61K 8/46, A61K 8/37, A61Q 19/10, A61Q 19/00

(54) **KOSMETISCHER SCHAUM AUS EINER EMULSION ENTHALTEND NATRIUMCETEARYLSULFAT UND GLYCERYLMONOSTEARAT**
COSMETIC FOAM EMULSION CONTAINING SODIUMCETEARYLSULFATE UND GLYCERYLMONOSTEARATE
EMULSION DE MOUSSE COSMETIQUE CONTENANT SODIUM CÉTÉARYLSULFATE ET GLYCÉRYLMONOSTEARATE

(30) Priorität: 04.11.2015 DE 102015221566
(43) Veröffentlichungstag der Anmeldung: 12.09.2018
(73) Patentinhaber: Beiersdorf AG, 20253 Hamburg (DE)
(72) Erfinder: SKUBSCH, Kerstin, 25497 Prisdorf (DE); MÜLLER, Claudia, 25499 Tangstedt (DE); RUPP, Katrin, 22417 Hamburg (DE); ECKERT, Julia, 22303 Hamburg (DE); SCHULZ, Sabine, 22159 Hamburg (DE)
(86) Internationale Anmeldenummer: PCT/EP2016/074833
(87) Internationale Veröffentlichungsnummer: WO 2017/076615

(56) Entgegenhaltungen:
- EP-A1- 2 179 986
- DE-A1- 10 361 202

## Beschreibung

Die vorliegende Erfindung betrifft einen kosmetischen Schaum aus einer Emulsion enthaltend Natriumcetearylsulfat und Glycerylmonostearat SE und einem die Emulsion aufschäumenden Gas oder Gasgemisch aus Propan, n-Butan und/oder Isobutan, wobei die Emulsion frei ist von Polyethylenglycol-Derivaten (PEG-Derivaten). Die Erfindung betrifft ferner eine Aerosoldose mit Entnahmeventil enthaltend einen derartigen Schaum.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen (ebenso wie einige Hautreinigungsprodukte) in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Neben der "klassischen" Anwendung, Emulsionen direkt aus dem Vorratsbehdftnis auf die Haut aufzutragen, gibt es auch eine geringere Anzahl von Anwendungen, bei denen die Emulsion zur Anwendung auf der Haut mit Hilfe eines Treibgases aufgeschäumt wird. Aufgeschäumte Emulsionen weisen aufgrund ihrer Schaumkonststenz ein besonderes Hautgefühl auf, dass von den Anwendern als besser auf der Haut verteilbar wahrgenommen wird, mit einem leichteren und weniger klebrigen Hautgefühl. Die Anwendung der moussigen Textur macht den Anwendern außerdem Spaß.

Nachteilig am Stande der Technik ist jedoch der Umstand, dass man für die Herstellung kosmetischer Emulsionsschäume den Zubereitungen Polyethylenglycole und/oder Polyethylenglycol-Derivate (PEG-Derivate) zusetzen muss (dies sind Verbindungen mit Alkohol- oder Säurefunktion, die mit Polyethylenglycolen ganz oder teilweise verethert oder verestert sind), um den Schaum über einen längeren Zeitraum stabil zu halten. Diese PEG-Derivate sind beim Verbraucher zunehmend unerwünscht, da ihnen von einigen Wissenschaftlern die gesundheitliche Unbedenklichkeit abgesprochen wird. Ob diese Bedenken wissenschaftlich begründet sind, kann im Rahmen der vorliegenden Erfindung dahingestellt bleiben. Tatsache ist hingegen, dass der Verbraucher zunehmend nach Kosmetika verlangt, die "PEG frei" sind.

Es war daher die Aufgabe der vorliegenden Erfindung, einen sensorisch attraktiven, über einen längeren Zeitraum stabilen kosmetischen Schaum auf der Basis einer aufgeschäumten Emulsion zu entwickeln, die "PEG frel" ist

Überraschend gelöst wild die Aufgabe durch einen kosmetischen Schaum gemäß Anspruch 1.

Der erfindungsgemäße Schaum ist überraschend gleichmäßig feinblasig. Er bildet ein so genanntes Mousse. Die Zubereitung zieht überraschend schnell in die Haut ein und hinterlässt ein angenehmes Hautgefühl.

Zwar kennt der Stand der Technik die EP1277455, EP1014916 und die DE 10138495, doch konnten diese Schriften nicht den Weg zur vorliegenden Erfindung weisen.
Ferner kennt der Fachmann die DE 103 61 202 und EP2 179 986, die ebenfalls nicht den Weg zur vorliegenden Erfindung weisen konnten.

Es ist erfindungsgemäß, wenn die Emulsion frei ist von Seifen (wobei eine Emulsion erfindungsgemäß als "frei von Seifen" gilt, wenn sie weniger als 0,2 Gewichts-%, bezogen auf das Gesamtgewicht der Emulsion an Seifen enthält). Dabei werden unter "Seifen" erfindungsgemäß Natrium- oder Kaliumsalze von Fettsäuren verstanden.

Es ist erfindungegemäß ebenfalls von besonderem Vorteil, wenn die Emulsion frei ist von Tensiden mit einem HLB-Wert von größer 10, wobei Natriumcetearylsulfat erfindungsgemäß nicht zu den Tensiden gezählt wird, sondern ein erfindungswesentticher Bestandteil ist

Dieser Sachverhalt ist umso überraschender, da Seifen und Tenside üblicherweise zur Stabilisierung von kosmetischen Schäumen erforderlich sind. Hingegen führt bei den erfindungsgemäßen Schäumen das Weglassen der Tenside hingegen zu einer Stabilisierung des Schaums und zu einem deutlich cremigeren Schaum, der einfacher zu verteilen ist.

In dieser erfindungegmäß vorteilhaften Ausführungsform sind die erfindungagernäßen Schäume überraschend stabiler und cremiger als unter Zusatz dieser oberflächenaktiven Verbindungen und verfügen über ein signifikant geringeres Hautreizungspotential.

Ein weiterer erfindungsgemäßer Vorteil dieser Formulierung ist die lange Lagerstabilität des Füllgutes (d.h. der Emulsion). Für den HersteUprozess ist es von Vorteil, eine lagerstabile Formulierung zu verwenden, teils das Füllgut nicht sofort in der Aerosolabfüllung abgefüllt werden kann. Es braucht bei einer späteren Abfüllung nicht umständlich aufgerührt werden. Bei der späteren Anwendung ist der Schaum einer lagerstabilen Emulsion in der Aerosoldose einfacher aufzuschütteln, was für eine gleichmäßige Verteilung des Treibgases in der Emulsion von Bedeutung ist. Nicht zuletzt ist so gewährleistet, dass die komplette Füllmenge gut zu entnehmen Ist und nicht durch Fehlanwendungen, die Treibgasmenge vorschnell "verbraucht" wird.

Es ist erfindungsgemäß vorteilhaft, wenn die erfindungsgemäße Emulsion Natriumcetearylsulfat in einer Menge von 0,1 bis 2 Gew.-%, bezogen auf das Gesamtgewicht der Emulsion-und erfindungsgemäß bevorzugt in einer Menge von 0,1 bis 0,5 Gew.-%, bezogen auf das Gesamtgewicht der Emulsion, enthält

Es Ist erfirtdungsgemäß vorteilhaft, wenn die erfindungsgemäße Emulsion Glycerylmonostearat SE in einer Menge von 0,1 bis 5 Gew.-%, bezogen auf das Gesamtgewicht der Emulsion und erfindungsgemäß bevorzugt in einer Menge von 1 bis 3 Gew.-%, bezogen auf das Gesamtgewicht der Emulsion, enthält.

Erfindungsgemäß wird selbstemulgierendes Glycerylmonostearat mit der INCI Glyceryl Monostearate SE eingesetzt.

Erfindungsgemäß vorteilhaft beträgt das Gewichtsverhältnis in der Emulsion von Natriumcetearylsulfat zu Glycerylmonostearat von 1:1 bis 1:20.

Erfindungsgemäß vorteilhafte Ausführungsformen der vorliegenden Erfindung sind dadurch gekennzeichnet, dass der Schaum gebildet wird aus 90-96 Gew.-% Emulsion und 4 bis 10 Gew.-% Gas bzw. Gasgemisch. Erfindungsgemäß bevorzugte Ausführungsformen sind dadurch gekennzeichnet, dass der Schaum gebildet wird aus 92-94 Gew.-% Emulsion und 6 bis 8 Gew.-% Gas bzw. Gasgemisch. Dass sich die erfindungsgemäßen Schäume bereits mit einer derartig geringen Menge an Gas gleichmäßig aufschäumen lassen, war dabei für den Fachmann nicht vorhersehbar. Die Ursache dafür ist die überraschend gute und einfache gleichmäßige Verteilung des Gases in der Emulsion.

Es ist erfindungsgemäß vorteilhaft, wenn als Gas ein Gasgemisch aus Butan, Iso-Butan und/oder Propan eingesetzt wird. Das Mischungsverhältnis der Gase variiert je nach Druckstufe z.B.:
Druckstufe 2,7 bar: 60 % Butan, 20% Propan und 20 % Iso-Butan.
Druckstufe 3,0 bar: 5,3 % Butan, 15,3% Propan und 79,4 % Iso-Butan.
Druckstufe 3,5 bar: 5 % Butan, 23% Propan und 72 % Iso-Butan.
Erfindungsgemäß bevorzugt sind die Druckstufen 2,7 bar, 3,0 bar und 3,5 bar.
Besonders bevorzugt sind die Druckstufen 3,0 bar und 3,5 bar.

Es ist erfindungsgemäß besonders vorteilhaft, wenn die Emulsion Ethanol enthält.
Dabei ist ein Ethanolgehalt von 1 bis 5 Gew.-%, bezogen auf das Gesamtgewicht der Emulsion erfindungsgemäß bevorzugt.

In diesem Zusammenhang war es besonders überraschend, dass der Schaum trotz Ethanolgehalt in der Emulsion stabil bleibt, da es zum allgemeinen Fachwissen gehört, dass Ethanol Schäume normalerweise destabilisiert. Darüber hinaus weist diese Kombination aus Schaum und Ethanol den überraschenden Vorteil auf, dass der Schaum trotz Gas und Ethanolgehalt nicht entflammbar ist, so dass die üblichen Sicherheitsvorkehrungen für solche Produkte bei der Lagerung, dem Transport und der Anwendung entbehrlich sind.

Es ist erfindungsgemäß vorteilhaft, wenn die Emulsion frei ist von Parabenen, Isothiazolinonen und 3-Iodpropargyl-*N*-butylcarbamat (IPBC).

Hingegen sind erfindungsgemäß vorteilhafte Ausführungsformen dadurch gekennzeichnet, dass die Emulsion Pirocton-Olamin (1-Hydroxy-4-methyl-6-(2,4,4-trimethylpentyl)- 2(1*H*)-pyridon monoethanolaminsalz) und/oder Phenoxyethanol enthält.

Enthält die Emulsion Pirocton-Olamin so ist es erfindungsgemäß von Vorteil, wenn der Gehalt an Pirocton-Olamin von 0,01 bis 1 Gew.-%, bezogen auf das Gesamtgewicht der Emulsion beträgt.

Enthält die Emulsion Phenoxyethanol, so ist es erfindungsgemäß von Vorteil, wenn der Gehalt an Phenoxyethanol von 0,1 bis 1 Gew.-%, bezogen auf das Gesamtgewicht der Emulsion beträgt.

Erfindungsgemäß vorteilhafte Ausführungsformen der vorliegenden Erfindung werden auch dadurch erhalten, dass die Emulsion EDTA und/oder Betain enthält.

Enthält die Emulsion EDTA so ist es erfindungsgemäß von Vorteil, wenn der Gehalt an EDTA von 0,01 bis 2 Gew.-%, bezogen auf das Gesamtgewicht der Emulsion beträgt.

Enthält die Emulsion Betain, so ist es erfindungsgemäß von Vorteil, wenn der Gehalt an Betain von 0,001 bis 6 Gew.-%, bezogen auf das Gesamtgewicht der Emulsion beträgt.

Erfindungsgemäß vorteilhafte Ausführungsformen der vorliegenden Erfindung sind dadurch gekennzeichnet, dass die Lipidphase der Emulsion einen oder mehrere Fettalkohole, Mandelöl, Kakaobutter und/oder Sheabutter enthält. Dabei ist es insbesondere überraschend gewesen, dass die Emulsionen, wenn sie Stearylalkohol bzw. Behenylalkohol enthalten, zu leicht und gleichmäßig mit dem Gas vermischbaren Zubereitungen führen, da beide Fettalkohole zu einer Erhöhung der Viskosität der Emulsion führen, was die Vermischbarkeit mit dem Gas(-gemisch) grundsätzlich erschweren sollte.

Die erfindungsgemäß vorteilhafte Einsatzkonzentration für Stearylalkohol beträgt von 0,01 bis 4 Gew.-%, bezogen auf das Gesamtgewicht der Emulsion.

Die erfindungsgemäß vorteilhafte Einsatzkonzentration für Behenylalkohol beträgt von 0,01 bis 4 Gew.-%, bezogen auf das Gesamtgewicht der Emulsion.

Die erfindungsgemäß vorteilhafte Einsatzkonzentration für Mandelöl beträgt von 0,01 bis 5 Gew.-%, bezogen auf das Gesamtgewicht der Emulsion.

Die erfindungsgemäß vorteilhafte Einsatzkonzentration für Kakaobutter beträgt von 0,01 bis 5 Gew.-%, bezogen auf das Gesamtgewicht der Emulsion.

Die erfindungsgemäß vorteilhafte Einsatzkonzentration für Sheabutter beträgt von 0,01 bis 5 Gew.-%, bezogen auf das Gesamtgewicht der Emulsion.

Erfindungsgemäß vorteilhafte Ausführungsformen der vorliegenden Erfindung sind dadurch gekennzeichnet, dass der Gewichtsanteil der Lipidphase an der Emulsion 7 bis 25 Gew.-%, bezogen auf das Gesamtgewicht der Emulsion, beträgt.

Es ist erfindungsgemäß von Vorteil, wenn die Emulsion weder Mineralöl noch Silikonöl enthält.

Silikonöle können jedoch auch in Mengen von kleiner 1 Gew.-%, bezogen auf das Gesamtgewicht der Emulsion enthalten sein.

Es ist vorteilhaft im Sinne der vorliegenden Erfindung, wenn der kosmetische Schaum dadurch gekennzeichnet ist, dass die Emulsion frei ist von Verdickungsmitteln wie z.B. Siliciumdioxid, Aluminiumsilikate, Polysaccharide bzw. deren Derivate, z. B. Hyaluronsäure, Xanthangummi, Hydroxypropylmethylcellulose, Polyacrylate, wie Carbopole, beispielsweise Carbomere und Acrylates/C10-30 Alkyl Acrylate Crosspolymer.

Die Wasserphase der erfindungsgemäßen Emulsion kann übliche kosmetische Hilfsstoffe enthalten, wie beispielsweise Glycerin, Elektrolyte, Selbstbräuner etc..

Erfindungsgemäß bevorzugt ist es dabei, wenn der kosmetische Schaum dadurch gekennzeichnet ist, dass die Emulsion, bezogen auf das Gesamtgewicht der Emulsion 5 bis 15 Gew.-% Glycerin enthält.

Darüber hinaus kann die erfindungsgemäße Emulsion vorteilhaft Salze enthalten, insbesondere Meersalz.

Es ist erfindungsgemäß vorteilhaft, wenn die erfindungsgemäße Emulsion einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Camosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivate, Glycyrrhiza Inflata Root Extract, Magnolienextrakt enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Erfindungsgemäß vorteilhafte Ausführungsformen der vorliegenden Erfindung sind dadurch gekennzeichnet, dass die Emulsion einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bomylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher, Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)esten; 4-(Dimethylamino)benzoesäure-amylester, 4-Methoxybenzalmalon-säuredi(2-ethylhexyl)esten 4-Methoxyzimtsäure(2-ethylhexyl)ester, 4-Methoxyzimtsäureisoamylester, 2-Hydroxy-4-methoxybenzophenon, 2-Hydroxy-4-methoxy-4'-methylbenzophenon; 2,2'-Dihydroxy-4-methoxybenzophenon; 4-(tert.-Butyt)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer, 4-(tert.-Butyl)-4'-methoxydibenzoylmethan; 2-(4'-Diethylamino-2'-hydoxybenzoyl)-benzoesäurehexylester, Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß besonders bevorzugt, wenn die Emulsion frei ist von Propyl- und Butylparaben, 3-lod-2-propinylbutylcarbamat, 3-(4-Methylbenzyliden)-campher, 2-Hydroxy-4-methoxybenzophenon (Oxybenzon) und halogenhaltigen Verbindungen.

Die erfindungsgemäßen kosmetischen Emulsionen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben und/oder Aerosile® (CAS-Nr. 7631-86-9) und /oder Talkum, Polymethylsilsesquioxane, Nylon, Silica Dimethyl Silyate

Erfindungsgemäß bevorzugt ist es, wenn der kosmetische Schaum dadurch gekennzeichnet ist, dass die Emulsion mit Polymethylsilsequioxan modifizierte Tapiokastärke (INCI Tapioca starch + Polymethylsilsesquioxane) enthält. Diese wird erfindungsgemäß vorteilhaft in einer Konzentration von 0,1 bis 3 Gew.-%, bezogen auf das Gesamtgewicht der Emulsion eingesetzt.

Der erfindungsgemäße Schaum bzw. die erfindungsgemäße Emulsion haben erfindungsgemäß vorteilhafte einen pH-Wert von 5 bis 7,5.

Erfindungsgemäß vorteilhaft wird der erfindungsgemäße Schaum in einer Aerosoldose mit Entnahmeventil aufbewahrt und aus dieser heraus angewendet. Zur Anwendung wird die erfindungsgemäße Mischung aus Emulsion und Gas(en) durch Schütteln in der Aerosoldose zuerst durchmischt und anschließend über das Entnahmeventil entnommen und auf die Haut appliziert. Dabei zeigt sich ein weiterer erfindungsgemäßer Vorteil, der darin liegt, dass der erfindungsgemäße Schaum sich im Vergleich zu herkömmlichen Schäumen vollständiger aus der Aerosoldose entnehmen lässt, d.h. dass die so genannte "Resteentleerung" überraschend höher ist als bei vergleichbaren, herkömmlichen Schäumen. Darüber hinaus zeigt der erfindungsgemäße Schaum auch bei niedrigen pH-Werten eine überraschend gute Packmittel-Kompatibilität, d.h. dass beispielsweise Korrosionseffekte deutlich seltener und schwächer ausfallen als bei vergleichbaren, herkömmlichen Systemen.

Als Aerosoldosen mit Entnahmeventil können die üblichen für kosmetische Zwecke bekannten Aerosoldosen-Systeme eingesetzt werden.

Erfindungsgemäß ist daher auch eine Aerosoldose mit Entnahmeventil enthaltend den erfindungsgemäßen Schaum, sowie ein Verfahren zur Anwendung kosmetischer Schäume auf der Haut, welches dadurch gekennzeichnet ist, dass die erfindungsgemäße Mischung aus Emulsion und Gas(en) durch Schütteln in der Aerosoldose zuerst durchmischt und anschließend über das Entnahmeventil entnommen und auf die Haut appliziert wird.

Nicht zuletzt ist es erfindungsgemäß bevorzugt, wenn der erfindungsgemäße Schaum dadurch gekennzeichnet ist, dass er in einer Aerosoldose mit Entnahmeventil enthalten ist.

### Vergleichsversuch

| | **A** | **B** |
|---|---|---|
| **Zusammensetzung** | **m [%]** | **m [%]** |
| Isopropyl Palmitat | 3,00 | 3,00 |
| Caprylic/Capric Triglycerid | 3,00 | 3,00 |
| Wasser | 74,40 | 74,40 |
| Natrium Cetearyl Sulfat | 0,15 | |
| Glyceryl Stearate SE | 1,50 | |
| PEG-40 Stearat | | 1,65 |
| Phenoxyethanol | 0,50 | 0,50 |
| Dimethicon | 1,00 | 1,00 |
| Behenyl Alkohol | 2,00 | 2,00 |
| Glycerin | 9,30 | 9,30 |
| 45% Natronlauge | 0,05 | 0,05 |
| Butyrospermum Parkii Butter | 1,00 | 1,00 |
| Carbomer | 0,10 | 0,10 |
| C15-19 Alkan | 4,00 | 4,00 |
| Formelstabilität nach 2 Monaten Lagerung bei 40°C | stabil | deutliche Wasserabscheidung (5 mm) |
| Befüllung in 200 ml Dose | 94% Emulsion | 94% Emulsion |
| Treibmittel | 6% Propan/Butan/ iso-Butan 3,5 bar* | 6% Propan/Butan/ iso-Butan 3,5 bar* |
| Aussehen des Schaumes | sehr cremig, feinblasig, stabil | wässrig, leer, mittelblasig, schnell zerfallend |

| | | |
|---|---|---|
| *= Druckgasstufe, siehe oben | | |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **C** | **D** | **E** | **F** |
|---|---|---|---|---|
| **Zusammensetzung in %** | **m [%]** | **m [%]** | **m [%]** | **m [%]** |
| Natrium Cetearyl Sulfat | 0,15 | 0,2 | 0,1 | 0,15 |
| Glyceryl Stearate SE | 1,5 | 1 | 2 | 1,5 |
| Isopropyl Stearat | 1 | | | 1 |
| CapryliclCapric Triglycerid | 3 | 6 | 3 | 3 |
| Octyldodecanol | 4 | | 3 | 4 |
| Isopropyl Palmitat | | 3 | 3 | |
| Mandelöl | 0,5 | | | |
| Dimethicon | | 1 | | |
| Shea Butter | 1 | 1 | 0,5 | 1 |
| Kakaobutter | | | 0,5 | |
| Tocopherolacetat | | | 0,2 | |
| Meersalz | | 0,1 | | |
| mit Polymethylsilsequioxan modifizierteTapiokastärke | 1 | | 1 | |
| Parfüm | 0,3 | 0,35 | 0,3 | 0,35 |
| Glycerin | 5 | 7 | 9 | 10 |
| Phenoxyethanol | 0,8 | 0,6 | 1,0 | 0,75 |
| Pirocton-Olamin | | 0,05 | | |
| Behenyl Alkohol | 2 | 2 | 2 | 2 |
| Ethanol | 1 | 2 | 3 | 1 |
| Natrium EDTA | 0,18 | 0,18 | 0,18 | 0,18 |
| Natronlauge/Zitronensäure q.s. zur pH Wert Einstellung | pH 5,5 | pH 7,0 | pH 7,0 | pH 6,5 |
| Wasser | ad 100 | ad 100 | ad 100 | ad 100 |
| Befüllung in 200 ml Dose | 94% Emulsion | 96% Emulsion | 95% Emulsion | 94% Emulsion |
| Treibmittel | 6% Propan/Butan/ iso-Butan 2,7 bar* | 4% Propan/Butan/ iso-Butan 3,0 bar* | 5% Propan/Butan/ iso-Butan 3,0 bar* | 6% Propan/Butan/ iso-Butan 3,5 bar* |

| | | | | |
|---|---|---|---|---|
| *Druckgasstufe, siehe oben | | | | |

| | **G** | **H** | **I** | **K** |
|---|---|---|---|---|
| **Zusammensetzung in %** | **m [%]** | **m [%]** | **m [%]** | **m [%]** |
| Natrium Cetearyl Sulfat | 0,15 | 0,15 | 0,15 | 0,15 |
| Glyceryl Stearate SE | 1,5 | 1 | 2 | 1,5 |
| Mineralöl | 3 | 2 | | 1 |
| Caprylic/Capric Triglycerid | 3 | | 3 | 3 |
| Octyldodecanol | 4 | | 3 | 4 |
| Isopropyl Palmitat | 3 | 3 | 3 | |
| Mandelöl | 0,5 | | | |
| Dimethicon | | 1 | | |
| Shea Butter | 1 | | | 1 |
| Vitamin C | 1 | | | |
| Betain | | 0,1 | 0,5 | |
| mit Polymethylsilsequioxan modifizierteTapiokastärke | 1 | | 1 | |
| Parfüm | 0,3 | 0,35 | 0,3 | 0,35 |
| Glycerin | 5 | 7 | 9 | 10 |
| Phenoxyethanol | 0,8 | 0,2 | | 0,75 |
| Stearyl Alkohol | 2 | 2 | 2 | 2 |
| Ethanol | 1 | 4 | 3 | 2 |
| Natrium EDTA | 0,18 | 0,18 | 0,18 | 0,18 |
| Natronlauge / Zitronensäure zur pH Wert Einstellung (quantum satis) | pH 6,5 | pH 7,0 | pH 7,0 | pH 6,5 |
| Wasser | ad 100 | ad 100 | ad 100 | ad 100 |
| Befüllung in 200 ml Dose | 95% Emulsion | 94% Emulsion | 94% Emulsion | 94% Emulsion |
| Treibmittel | 5% Propan/Butan/ iso-Butan 3,5 bar* | 6% Propan/Butan/ iso-Butan 3,5 bar* | 6% Propan/Butan/ iso-Butan 3,5 bar* | 6% Propan/Butan/ iso-Butan 3,5 bar* |

| | | | | |
|---|---|---|---|---|
| *Druckgasstufe, siehe oben | | | | |

## Patentansprüche

1. Kosmetischer Schaum aus
a) einer Emulsion enthaltend eine Kombination aus Natriumcetearylsulfat und Glycerylmonostearat SE und
b) einem die Emulsion aufschäumenden Gas oder Gasgemisch aus Propan, n-Butan und/oder Isobutan,
wobei die Emulsion frei ist von Polyethylenglycol-Derivaten (PEQ-Derivaten), **dadurch gekennzeichnet, dass** die Emulsion frei ist von Seifen und Tensiden mit einem HLB-Wert von größer 10, wobei Natriumcetearylsulfat nicht zu diesen Tensiden gezählt wird.

2. Kosmetischer Schaum nach Anspruch 1, **dadurch gekennzeichnet, dass** die Emulsion Natriumcetearylsulfat in einer Menge von 0,1 bis 2 Gew.-%, bezogen auf das Gesamtgewicht der Emulsion, enthält.

3. Kosmetischer Schaum nach einem der vorhergehenden Ansprüche, **dadurch gekennzeichnet, dass** die Emulsion Glycerylmonostearat SE in einer Menge von 0,1 bis 5 Gew.-%, bezogen auf das Gesamtgewicht der Emulsion, enthält,

4. Kosmetischer Schaum nach einem der vorhergehenden Ansprüche, **dadurch gekennzeichnet, dass** der Schaum gebildet wird aus 90-96 Gew.-% Emulsion und 4 bis 10 Gew.-% Gas bzw. Gasgemisch.

5. Kosmetischer Schaum nach einem der vorhergehenden Ansprüche, **dadurch gekennzeichnet, dass** die Emulsion frei ist von Verdickungsmitteln wie z.B. Siliciumdioxid, Aluminiumsltikate, Polysaccharide bzw. deren Derivate, z. B. Hyaluronsäure, Xanthangummi, Hydroxypropylmethylcellulose, Pölyacrylate, wie Carbopole, beispielsweise Carbomere und Acrylates/C10-30 Alkyl Acrylate Crosspolymer

6. Kosmetischer Schaum nach einem der vorhergehenden Ansprüche, **dadurch gekennzeichnet, dass** die Emulsion frei ist von Parabenen, Isothiazolinonen und 3-Iodpropargyl-*N*-butylosrbamat (IPBC).

7. Kosmetischer Schaum nach einem der vorhergehenden Ansprüche, **dadurch gekennzeichnet, dass** die Emulsion Ethanol enthält.

8. Kosmetischer Schaum nach einem der vorhergehenden Ansprüche, **dadurch gekennzeichnet, dass** die Emulsion Pirocton-Olamin (1-Hydroxy-4-methyl-6-(2,4,4-trimethylpentyl)-2(1*H*)-pyridon monoethanofaminsalz) und/oder Phenoxyethanol enthält,

9. Kosmetischer Schaum nach einem der vorhergehenden Ansprüche, **dadurch gekennzeichnet, dass** die Emulsion EDTA und/oder Betain enthält.

10. Kosmetischer Schaum nach einem der vorhergehenden Ansprüche, **dadurch gekennzeichnet, dass** die Lipidphase der Emulsion Stearylalkohol, Behenylalkohol, Mandelöl, Kakaobutter und/oder Sheabutter enthält.

11. Kosmetischer Schaum nach einem der vorhergehenden Ansprüche, **dadurch gekennzeichnet, dass** der Gewichtsanteil der Lipidphase an der Emulsion 7 bis 25 Gew.-%, bezogen auf das Gesamtgewicht der Emulsion beträgt

12. Kosmetischer Schaum nach einem der vorhergehenden Ansprüche, **dadurch gekennzeichnet, dass** die Emulsion weder Mineralöl noch Silikonöl enthält.

13. Kosmetischer Schaum nach einem der vorhergehenden Ansprüche, **dadurch gekennzeichnet, dass** die Emulsion mit Polymethylsilsequioxan modifizierteTapfokastärke (INCI Tapioca starch + Polymethylsilsesquioxane) enthält.

14. Kosmetischer Schaum nach einem der vorhergehenden Ansprüche, **dadurch gekennzeichnet, dass** die Emulsion, bezogen auf das Gesamtgewicht der Emulsion 5 bis 15 Gew.-% Glycerin enthält.

15. Kosmetischer Schaum nach einem der Ansprüche 1 bis 15, **dadurch gekennzeichnet, dass** er in einer Aerosoldose mit Entnahmeventil enthalten ist.

16. Aerosoldose mit Entnahmeventil enthaltend einen kosmetischen Schaum nach einem der Ansprüche 1 bis 15.

## Claims

1. Cosmetic foam composed of
a) an emulsion comprising a combination of sodium cetearyl sulfate and glyceryl monostearate SE and
b) a gas or gas mixture of propane, n-butane and/or isobutane which foams the emulsion,
wherein the emulsion is free of polyethylene glycol derivatives (PEG derivatives), **characterized in that** the emulsion is free of soaps and surfactants having an HLB value of greater than 10, wherein sodium cetearyl sulfate is not counted among these surfactants.

2. Cosmetic foam according to Claim 1, **characterized in that** the emulsion comprises sodium cetearyl sulfate in an amount from 0.1 to 2% by weight, based on the total weight of the emulsion.

3. Cosmetic foam according to either of the preceding claims, **characterized in that** the emulsion comprises glyceryl monostearate SE in an amount from 0.1 to 5% by weight, based on the total weight of the emulsion.

4. Cosmetic foam according to any of the preceding claims, **characterized in that** the foam is formed from 90-96% by weight emulsion and 4 to 10% by weight gas or gas mixture.

5. Cosmetic foam according to any of the preceding claims, **characterized in that** the emulsion is free of thickeners such as, for example, silicon dioxide, aluminum silicates, polysaccharides or derivatives thereof, e.g. hyaluronic acid, xanthan gum, hydroxypropylmethylcellulose, polyacrylates such as carbopols, for example carbomers and acrylates/C10-30 alkyl acrylate crosspolymer.

6. Cosmetic foam according to any of the preceding claims, **characterized in that** the emulsion is free of parabens, isothiazolinones and 3-iodopropargyl N-butylcarbamate (IPBC).

7. Cosmetic foam according to any of the preceding claims, **characterized in that** the emulsion comprises ethanol.

8. Cosmetic foam according to any of the preceding claims, **characterized in that** the emulsion comprises piroctone olamine (1-hydroxy-4-methyl-6-(2,4,4-trimethylpentyl)-2(1H)-pyridone monoethanolamine salt) and/or phenoxyethanol.

9. Cosmetic foam according to any of the preceding claims, **characterized in that** the emulsion comprises EDTA and/or betaine.

10. Cosmetic foam according to any of the preceding claims, **characterized in that** the lipid phase of the emulsion comprises stearyl alcohol, behenyl alcohol, almond oil, cocoa butter and/or shea butter.

11. Cosmetic foam according to any of the preceding claims, **characterized in that** the proportion by weight of the lipid phase of the emulsion is 7 to 25% by weight, based on the total weight of the emulsion.

12. Cosmetic foam according to any of the preceding claims, **characterized in that** the emulsion comprises neither mineral oil nor silicone oil.

13. Cosmetic foam according to any of the preceding claims, **characterized in that** the emulsion comprises tapioca starch modified with polymethylsilsesquioxane (INCI Tapioca starch + Polymethylsilsesquioxane).

14. Cosmetic foam according to any of the preceding claims, **characterized in that** the emulsion comprises 5 to 15% by weight glycerol, based on the total weight of the emulsion.

15. Cosmetic foam according to any of Claims 1 to 15, **characterized in that** said foam is present in an aerosol can with release valve.

16. Aerosol can with release valve comprising a cosmetic foam according to any of Claims 1 to 15.

## Revendications

1. Mousse cosmétique, constituée par :
a) une émulsion contenant une combinaison de sulfate de cétéaryle sodique et de monostéarate de glycéryle SE, et
b) un gaz ou mélange gazeux moussant l'émulsion, constitué par du propane, du n-butane et/ou de l'isobutane,
l'émulsion étant exempte de dérivés de polyéthylène glycol (dérivés de PEG), **caractérisée en ce que** l'émulsion est exempte de savons et de tensioactifs ayant une valeur HLB supérieure à 10, le sulfate de cétéaryle sodique n'étant pas compté parmi ces tensioactifs.

2. Mousse cosmétique selon la revendication 1, **caractérisée en ce que** l'émulsion contient du sulfate de cétéaryle sodique en une quantité de 0,1 à 2 % en poids, par rapport au poids total de l'émulsion.

3. Mousse cosmétique selon l'une quelconque des revendications précédentes, **caractérisée en ce que** l'émulsion contient du monostéarate de glycéryle SE en une quantité de 0,1 à 5 % en poids, par rapport au poids total de l'émulsion.

4. Mousse cosmétique selon l'une quelconque des revendications précédentes, **caractérisée en ce que** la mousse est formée par 90 à 96 % en poids d'émulsion et 4 à 10 % en poids de gaz ou de mélange gazeux.

5. Mousse cosmétique selon l'une quelconque des revendications précédentes, **caractérisée en ce que** l'émulsion est exempte d'épaississants tels que par exemple le dioxyde de silicium, les silicates d'aluminium, les polysaccharides et leurs dérivés, par exemple l'acide hyaluronique, la gomme de xanthane, l'hydroxypropylméthylcellulose, les polyacrylates, tels que les carbopols, par exemple les carbomères et Acrylates/C10-30 Alkyl Acrylate Crosspolymer.

6. Mousse cosmétique selon l'une quelconque des revendications précédentes, **caractérisée en ce que** l'émulsion est exempte de parabènes, d'isothiazolinones et de *N*-butylcarbamate de 3-iodopropargyle (IPBC).

7. Mousse cosmétique selon l'une quelconque des revendications précédentes, **caractérisée en ce que** l'émulsion contient de l'éthanol.

8. Mousse cosmétique selon l'une quelconque des revendications précédentes, **caractérisée en ce que** l'émulsion contient de la piroctone-olamine (sel de monoéthanolamine de 1-hydroxy-4-méthyl-6-(2,4,4-triméthylpentyl)-2(1*H*)-pyridone) et/ou du phénoxyéthanol.

9. Mousse cosmétique selon l'une quelconque des revendications précédentes, **caractérisée en ce que** l'émulsion contient de l'EDTA et/ou de la bétaïne.

10. Mousse cosmétique selon l'une quelconque des revendications précédentes, **caractérisée en ce que** la phase lipidique de l'émulsion contient de l'alcool stéarylique, de l'alcool béhénylique, de l'huile d'amande, du beurre de cacao et/ou du beurre de karité.

11. Mousse cosmétique selon l'une quelconque des revendications précédentes, **caractérisée en ce que** la proportion en poids de la phase lipidique de l'émulsion est de 7 à 25 % en poids, par rapport au poids total de l'émulsion.

12. Mousse cosmétique selon l'une quelconque des revendications précédentes, **caractérisée en ce que** l'émulsion ne contient ni de l'huile minérale, ni de l'huile de silicone.

13. Mousse cosmétique selon l'une quelconque des revendications précédentes, **caractérisée en ce que** l'émulsion contient de l'amidon de tapioca modifié avec du polyméthylsilsesquioxane (INCI : Tapioca starch + Polymethylsilsesquioxane).

14. Mousse cosmétique selon l'une quelconque des revendications précédentes, **caractérisée en ce que** l'émulsion contient 5 à 15 % en poids de glycérine, par rapport au poids total de l'émulsion.

15. Mousse cosmétique selon l'une quelconque des revendications 1 à 15, **caractérisée en ce qu'**elle est contenue dans une bombe aérosol munie d'une vanne de prélèvement.

16. Bombe aérosol munie d'une vanne de prélèvement contenant une mousse cosmétique selon l'une quelconque des revendications 1 à 15.
